# EUROPEAN PATENT SPECIFICATION

(11) **EP 2 796 114 B1**
(45) Date of publication and mention of the grant of the patent: **02.03.2016**
(21) Application number: 13733334.0
(22) Date of filing: 15.03.2013
(51) Int. Cl.: A61F 5/058

(54) **ELEMENT WITH VARIABLE STIFFNESS CONTROLLED BY NEGATIVE PRESSURE**
DURCH UNTERDRUCK GESTEUERTES ELEMENT MIT VARIABLER STEIFIGKEIT
ÉLÉMENT À RIGIDITÉ VARIABLE COMMANDÉE PAR PRESSION NÉGATIVE

(43) Date of publication of application: 29.10.2014
(73) Proprietor: Textia Innovative Solutions, S.L., 48160 Derlo (Bizkaya) (ES)
(72) Inventor: BUREAU, Maxime, 20009 Donostia-San Sebastián (ES); KELLER, Thierry, 20009 Donostia-San Sebastián (ES); VENEMAN, Jan F., 20009 Donostia-San Sebastián (ES); VERA MARTÍN, Carolina, 20009 Donostia-San Sebastián (ES)
(74) Representative: Stiebe, Lars Magnus
(86) International application number: PCT/ES2013/070177
(87) International publication number: WO 2014/140389

(56) References cited:
- WO-A1-2011/079865
- US-A- 5 093 388
- Sarah Yang: "08.22.2006 - Engineers create gecko-inspired high-friction micro-fibers", , 22 June 2006 (2006-06-22), XP055091012, Retrieved from the Internet: URL:http://www.berkeley.edu/news/media/rel eases/2006/08/22_microfiber.shtml [retrieved on 2013-12-02]

## Description

### TECHNICAL FIELD

The invention relates to an element with variable stiffness controlled through negative pressure, such as suction or vacuum. The present invention is applicable:
- to certain medical devices (such as casts, functional orthoses, insoles, and emergency medical devices such as splints for limbs and full body first aiders),
- to sporting goods (such as skates, ski boots, surfboards and protective equipment in sports such as helmets or knee and chest guards),
- to safety elements that rigidify in case of a collision or accident,
- to constructive elements for example to be used to make reconfigurable camping furniture or toys,
- to mould-elements for production of composites,
- to packaging elements, or
- to safety elements that rigidify in case of a collision or accident, for example, in the automotive field.

### STATE OF THE ART

It is known to use negative pressure such as suction or vacuum to provide a way to convert an element from a flexible state, in which the element is easily formed and can be adapted to conform a specific desired shape (such as a part of the human body), to a rigid state, in which the element is rigid and provides support, protection and/or stabilisation, and vice versa. The basic structure of devices employing negative pressure usually comprises inner fillers which are commonly movable particles and a flexible, air-impermeable thin outer cover. The structure normally enables the device to be easily and readily fitted around the body and affected limbs. When the device becomes the desired shape in the desired position, it is subjected to negative pressure and then atmospheric pressure compresses the flexible outer cover and applies substantial pressure to the entire mass of particles. The frictional force between the particles and the cover resist movement relative to each other, thereby providing rigidity. Usually a valve is included for sealing the cover when evacuated to maintain rigidity of the device. The soft state from the rigid state is usually obtained by opening the valve and whiff.

Several patents about orthopaedic devices employing negative pressure have been published. Patent document US 2005/0137513 discloses a structure to maintain a homogeneous thickness for devices for supporting and stabilizing an injured person or body parts. The device has an inner region enveloped by two flexible films and the inner region is divided into two insertion bodies which are respectively formed with two air-permeable, flexible material strips. Each insertion body is divided into chambers containing loose particles, by way of intersecting seams formed between the material strips. The seams on both insertion bodies are staggered in relation to each other in both directions in such a way that the particles combine to form a substantially homogeneously thick particle layer. However, such a structure made of granules or particles has the problem of being too thick, which leads to practical limitations such as transporting size, and a high volume leading to problems such as a long evacuation time to reach the desired negative pressure level.

In order to solve the problem of undesired thickness and volume, the body fitting element a with controlled fitness disclosed in patent document WO 2011/079865 is made of a gas tight envelope enclosing a plurality of layers and having a valve adapted to evacuate the interior of the envelope. Each layer is made of a core made of a material with a high Young modulus and flexibility coated on both sides with a cover layer made of a material with a high friction coefficient. However, such a body fitting element presents the following problems:
- Once vacuum has been applied and the body fitting element is in its rigid state, when the valve is opened to go to the flexible state, the fact is that the layers may keep stuck one to the other due to their tackiness, and the flexible state is thus not recovered properly.
- In the rigid state, when under a bending stress, the layers may delaminate (disconnect the coating from the core).
- The body fitting element disclosed in WO 2011/079865 included some strips made
of a material with a low friction coefficient, in order to properly recover the flexible state under atmospheric pressure. However, these strips reduce the effective friction surfaces, which consequently reduces the stiffness of the element in rigid state and thus affects its proper functioning.

In sum, an element with controllable stiffness is needed, which solves in an efficient way both the problems of tackiness and delamination, in the scenario described above.

### DESCRIPTION OF THE INVENTION

The present invention refers to an element with variable stiffness controlled by negative pressure according to claim 1. Preferred embodiments of the element are defined in the dependent claims.

It is an object of the present invention to provide an element with variable stiffness controllable by negative pressure which overcomes the tackiness and delamination problems of the existing elements with controllable stiffness. In order to do so, the element with variable stiffness controllable by negative pressure of the present invention is fully reversible between the flexible and rigid states, at the same time maintaining or even improving the stiffness ratio between the rigid and flexible state compared with the stiffness ratio of current solutions.

The element with variable stiffness controlled by negative pressure of the present invention comprises:
- a gas-tight envelope;
- a plurality of flexible layers in the envelope, each layer having a first surface and a second surface; and,
- a valve adapted to evacuate the interior of the envelope.

According to a first aspect of the invention the first and second surfaces of two adjacent layers:
- have a friction coefficient between each other which is higher than 0.5; and
- have adhesion properties, such that a normal force per unit area of no more than 0.07 N/mm² is required to separate them and/or the energy per unit area required to separate them in normal direction being below 6.7 J/m².

Thus, the element of the present invention has a laminar structure comprising several layers and has the following properties:
- high friction coefficient between layers due to the selection of materials used and the fact that the whole surface of the layers support friction; and,
- low adhesion between layers, especially when there is no normal force.

This way the first and second surfaces are slidable relative to each other under atmospheric pressure.

A further advantage of the present invention is that the layers can be made thinner than the layers of prior-art elements (which included coated core layers), due to the fact that the layers can be made of a single material or composite with the necessary properties of high friction coefficient and low adhesion properties, thereby eliminating the need for both coating layer and core, and the corresponding adhesive layer between them.

With this specific element configuration of the invention the stiffness ratio of the element between its flexible and rigid states is improved:
- firstly, because the layers are not stuck to each other at atmospheric pressure, which makes the element more flexible and easier to shape in its softer state; and
- secondly, since there is no need for strips of low friction or sewing, the friction surface is increased which makes the element stiffer in its rigid state.

Depending on the applications of the element, the layers can be made of a single material, or they can be made of a plurality of fibres embedded in a matrix. In both cases the layers can be made of a continuous or homogeneous sheet, or the layer can be made of a woven structure of tapes or ribbons.

Where the layers are made of a matrix reinforced with fibres, the fibres can be unidirectional, bidirectional or multidirectional. Only the composites having unidirectional fibres are suitable for the woven structure with tapes.

The fibres are preferably selected from fibre glass, carbon, aramid or polyester fibres. And the matrix is preferably made of a thermostable polymer or a thermoplastic polymer.

According to another preferred embodiment, the layers can comprise a core layer coated by at least one coating on one side of the core. Preferably the core is coated by respective first and second coatings, one coating on each side of the core.

Such coatings are preferably made of a thermoplastic polyurethane elastomer.

The adhesion properties of the layers are preferably measured using an adapted version of a standardised method, such as the ASTM D2979-01, "Standard test method for pressure-sensitive tack of adhesives using an inverted probe machine". Preferably the adhesion properties are measured using a probe-tack test with a pre-load equivalent to the atmospheric pressure and a waiting pre-load time of 100 s.

The friction coefficient is preferably measured using a standardized procedure such as the ASTM D 1894 "Standard test method for static and kinetic coefficients of friction of plastic film and sheeting".

In the case of layers being made of woven tapes, the adhesion properties can be measured using the adapted probe-tack test on a specific number of tapes placed adjacent to each other over a flat surface, such that the resulting width is higher than the minimum value set by the standard.

The adhesion properties of the layers are measured on layers as they are in its conditions of use; that is, although the standard indicates that the layers should be cleaned before any measuring is carried out, for measuring the adhesion properties of the layers of the element in the present invention the layers need not be previously cleaned; their adhesion properties should be measured in the same conditions as when they are in use inside the element.

Additional advantages and features of the invention will become apparent from the detailed description that follows and will be particularly pointed out in the appended claims.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
Figure 1 shows a sectional view of an element with variable stiffness according to the invention.
Figure 2 is a perspective view of a first embodiment of the layers inside the element.
Figures 3, 4, 5, 6 and 7 show perspective views of other possible embodiments of the layers inside the element.
Figure 8 is a perspective view of a section of an element according to the invention intended for medical applications.
Figure 9 is a top view of a ribbon weaving structure of the layers according to another embodiment of the element.
Figure 10 schematically shows the adapted method used in the present case to measure adhesion of the layers.

### DESCRIPTION OF A PREFERRED EMBODIMENT

The following description is not to be taken in a limiting sense but is given solely for the purpose of describing the broad principles of the invention. Next embodiments of the invention will be described by way of example, with reference to the above-mentioned drawings showing elements and results according to the invention.

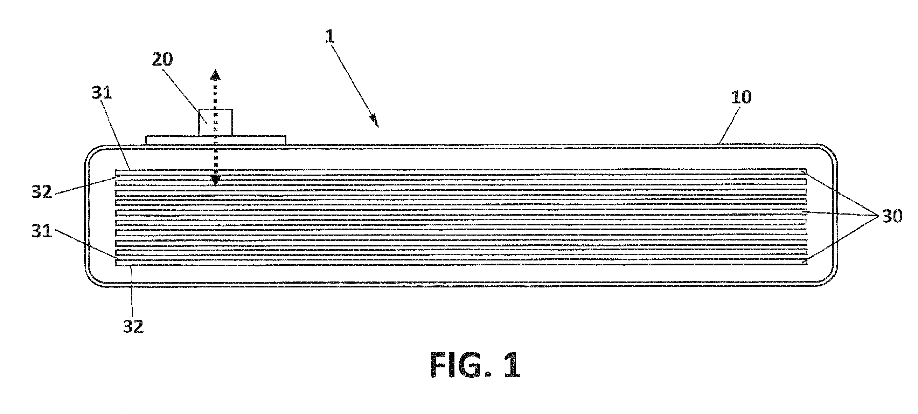

Figure 1 shows a preferred embodiment of an element 1 with variable stiffness according to the invention. The element 1 comprises of a hermetic stretchable envelope 10 enclosing a plurality of flexible layers 30 and a valve 20 adapted to evacuate the interior of the envelope. The gas tight envelope 10 is suitable for being subjected to a controlled pressure, and has a valve 20 adapted to evacuate the interior of the envelope.

In a known manner, when atmospheric pressure is inside the envelope 10, the layers 30 are uncompressed. When vacuum is applied, the flexible layers 30 are compressed together increasing the friction between them, which in turn increases the stiffness of the element 1 as a whole. This way the element 1 has variable state possibilities, from a soft, flexible state at atmospheric pressure to a rigid state when depressurized.

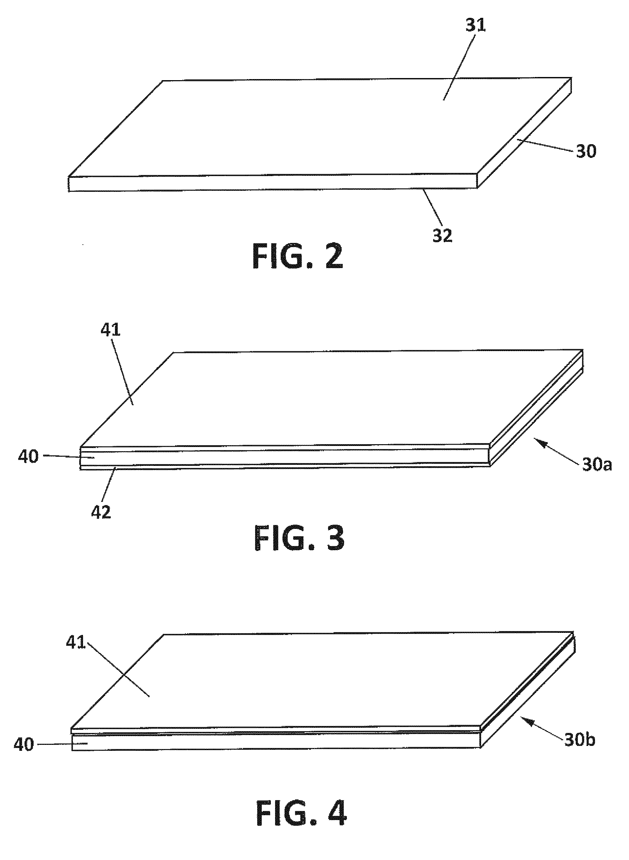

The novelty of the present element 1 resides in the specific structure and material of the layers 30, as shown in Figure 2; each layer 30 is made of a single material or composite has a first surface 31 on one side and a second surface 32 on the other side.

In order that the element 1 works properly and its flexible or soft state is adequately recovered once vacuum has been applied and afterwards released, it is important that the first and second surfaces 31, 32 of the layers are made of such materials, thickness and surface finish that result in both a high friction coefficient and low adhesion connection.

The layers are such that the friction coefficient between the first surface 31 and the second surface 32 of two adjacent layers 30 is higher than that of materials normally used for lubrication. The friction coefficient between the surfaces is above 0.6. More preferably, the friction coefficient is above 1.

In order to measure the friction coefficient a standardized procedure can be used, such as the ASTM D 1894 "Standard test method for static and kinetic coefficients of friction of plastic film and sheeting".

An essential requisite of the layers is that they have low adhesion properties. By low adhesion it is meant that the tangential adhesion force between the first and second surfaces 31, 32 of two adjacent layers 30 is such that the layers 30 slide relative to each other when there is no normal force. In fact, the first and second surfaces of the layers have low adhesion properties such that a normal force per unit area of below 0.07 N/mm² is required to separate them (that is, a compressive normal force of -0.07 N/mm²), and/or the energy per unit area required to separate them in normal direction being below 6.7 J/m².

This is an important feature to prevent the different layers to remain stuck to each other once the vacuum is no longer applied and the element 1 recovers its flexible state.

It should be considered that the friction and tackiness properties of the layers are not only influenced by the material of the layer, but also by the thickness and the surface finish (roughness Ra) of the layer. This is why in order to characterise the interface between the first and second surfaces 31, 32 of two adjacent layers 30 the friction and tackiness tests are made on two flexible layers 30 to take into account the effect of the manufacturing processes.

The adhesion properties have been measured by means of an adapted probe-tack test with a pre-load equivalent to the atmospheric pressure and a waiting pre-load time of 100s, which is the maximum timescale mentioned in the norm for adhesive properties to settle.

A standardised method to measure adhesion of adhesives is described by ASTM D2979-01, "Standard test method for pressure-sensitive tack of adhesives using an inverted probe machine". In the present case an adapted version of this method has been used. The standardised method had to be modified since adhesion between layers which are not adhesives is being measured; these layers have a lower adhesion force, and where the adhesion depends on the material, thickness and roughness of the layers. The layers can be cleaned with alcohol (or any other means) prior to the measuring, as indicated in the standard. But it is possible to take the measurements without previously cleaning the surface of the layers, in order to not alter the adhesion properties of the layers on their conditions of use.

What is important is that the standardised method is repeated several times, so as to have a statistically significant number of measurements, such that it is possible to disregard outlier values.

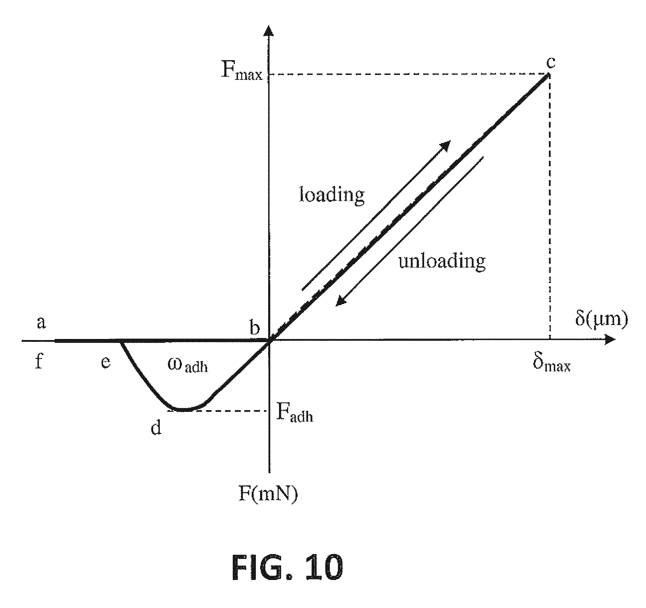

The measuring method is schematically shown in Figure 10, where force (F) is represented with respect to displacement (δ) between the layers. The adhesion measurement method is as follows:
a-c) The surfaces of the layers are approached at a constant speed, and at some point contact each other and are compressed until compressive force Fₘₐₓ is reached.
c-f) The surfaces of the layers are separated at a constant speed of 5 mm/s according to the standard.

More specifically:
a-b) The surfaces move towards each other at constant speed, there being no contact between them.
b-c) Once there is contact between the surfaces, a compression force is built up by the motion, until it reaches its peak value of Fₘₐₓ. Then movement between the layers stops, and the force is kept at the constant level Fₘₐₓ.
c-d) The surfaces are moving out of each other at constant speed. Initially, the compression force is removed, until a point where an adhesion force between the layers builds up until a maximum value F_{adh} is reached. This adhesion force has a direction opposite to the initial compressive force.
d-e) The adhesion force between the layers disappears through disconnection of surfaces.
e-f) The surfaces are separated from each other without contact, at a constant speed.

The specific adaptations to the standardised test are:
- A circular contact surface of 50 mm is used, instead of the 5 mm probe of the standard, because of lower adhesion forces. In the case of an embodiment comprising tapes, a number of tapes should be lined up next to each other, so that lined up together they cover the circular surfaces of 50 mm.
- A normal upright probe machine is used instead of an inverted probe machine, because of lower adhesive forces.
- The test of adhesion is carried out between the actual layers of the element, instead of carrying out the test between a stainless steel probe and the adhesive.
- The constant speed approaching movement is stopped when the value Fₘₐₓ is reached; at this point the probe machine is programmed to keep the static load constant at Fₘₐₓ. The value of Fₘₐₓ used is 200 N, which corresponds to a compressive load in the order of magnitude of the atmospheric pressure on the surface.
- The probe is lifted vertically upwards from a resting surface during steps c-f.
- The static load is maintained for 100 s (instead of 1 s).
- Real layers are used in the test, instead of the specified adhesive surface layer thickness specified in the norm.
- Values are averaged over at least five measurements.
- The adhesion is characterised by:
   - by F_{adh}/A_{surface}, where F_{adh} is the maximum force measured while disconnecting the surfaces and A_{surface} is the area of the contact surface, and
   - by ω_{adh}/A_{surface}, where ω_{adn} is the energy required to disconnect the two layers.

The following Table 1 shows examples of layers, the adhesion properties of which have been measured with the adapted probe-tack test just explained.

**Table 1**

| Specification of layer (material(s), thickness of layer) | Minimum tack stress (N/mm²) | Tack Energy (J/m²) |
|---|---|---|
| Fibreglass fabric (204 g/m²) embedded in TPU Epurex® 4201 AU, 250 µm | 0.0259 | 2.2669 |
| TPU Epurex® 4201 AU, 75 µm (at conditions of use) | 0.0424 | 0.8481 |
| Fibreglass fabric (125 g/m²) TPU BASF® 890 A10, 200µm | 0.0222 | 1.2159 |
| Fibreglass fabric (125 g/m²) TPU BASF® S 85 A11, 200µm | 0.0565 | 6.8287 |

A preferred embodiment of the element 1 of the invention example I, having the flexible layers 30 of Figure 2 includes thirty-seven layers 30, each layer having a thickness of 80 µm. Each layer is made of thermoplastic polyurethane, in this case, of Epurex® 4201 AU (supplied by Epurex Films GmbH). The resulting element allows switching between a rigid state with a Young's modulus of 167 MPa (obtained at a negative pressure of -0.86 bar) to a flexible state with a Young's modulus of 22 MPa (measured at atmospheric pressure). The Young's modulus was obtained for a strain of 0.2%-0.4%.

For certain applications it is necessary that the element of the invention has a certain degree of stiffness. In order to obtain such stiffness, the layers of the element can be improved as shown in any of Figures 3-7.

Figure 3 shows another possible embodiment of the layer 30a. In this case the layer 30a comprises a core 40 coated by a first coating 41 on one side and a second coating 42, which first and second coatings constitute the first and second surfaces -of the layer. Usually the first and second coatings 41, 42 are glued to respective sides of the core 40.

The core 40 is essentially a continuous sheet of a flexible material having a high Young's modulus. Having a high Young's modulus means that the Young's modulus of the core is higher than the Young's modulus of the materials used because of their elasticity (such as rubbers). Besides, the Young's modulus of the material constituting the core 40 is higher than Young's modulus of the material of the coatings 41, 42. The material forming the core 40 preferably has a Young's modulus above 0.2 GPa, such as LDPE, which provides the element with stiffness valid for certain applications.

Preferably the Young's modulus of the core 40 material is higher than 0.8 GPa.

The core can be made of any of the following materials:
- Thermoplastics such as ABS, PEEK, PP, PEHD or PVC.
- Metals such as aluminium, brass, or iron.
- Wood, paper.

It is possible that the first and second coatings 41, 42 on each side of the core 40 are made of the same material, including specific layer thickness and surface finish, resulting in high friction coefficient and low adhesion properties, as is the case of the layer shown in Figure 3. It is also possible that they are made of different materials, including specific layer thickness and surface finish, such that each of the layers has the corresponding high friction and low adhesion properties when in mutual contact.

Figure 4 shows another possible layer 30b for the element of the invention. In this case the layer 30b comprises a core 40 and a first coating 41 only on one side of the core 40. The core 40 is made of a material having a high Young's modulus, and the coating 41 is made of a material with a lower Young's modulus. The thickness of the core 40 is higher than the thickness of the first coating 41. Also, in order to fully achieve the properties of low adhesion and high friction between the layers 30b in this embodiment, the coating 41 has a smooth surface finish while the surface finish of the core 40 is rough.

Additionally, in the embodiment of the layer 30a shown in Figure 3, it is important that the tangential adhesion force between the layers 30a, that is, the tangential adhesion force between the coatings 41, 42 of two adjacent layers (which are the surfaces in contact), is lower than the maximum tangential adhesion force due to the gluing between the coatings 41, 42 and the core 40 in each layer 30a. This is also an important feature since otherwise delamination of the layers may occur during flexion solicitation.

Similarly, in the embodiment of the layer 30b of Figure 4, it is important that the tangential adhesion force between the layers 30b, that is, the tangential adhesion force between the core 40 of one layer 30b and the coating 41 of the adjacent layer 30b (which are the surfaces in contact), is lower than the maximum tangential adhesion force due to the gluing between the coating 41 and the core 40 in each layer 30b.

Suitable materials for the coatings are: some thermoplastic polyurethanes, Acronal/Styrofan resin (40% of Acronal® 12 DE with 60% of Styrofan® D422, from BASF), polyurea resin, silicone, rubber, silicone rubber, latex.

To further improve the stiffness properties of the element 1 for those applications that may require it (such as construction elements), the layers can comprise a matrix reinforced with fibres.

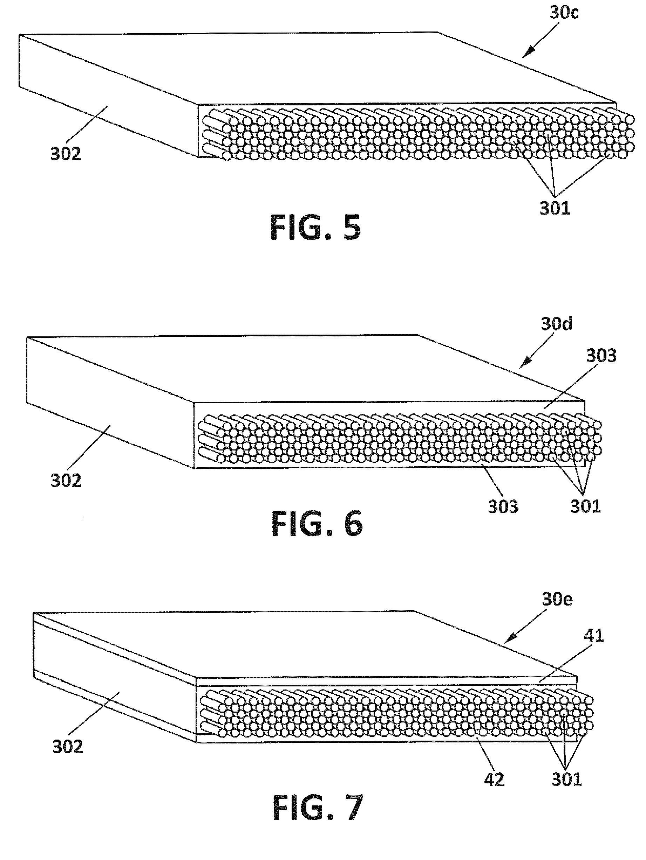

As shown in Figure 5, the layer 30c comprises a plurality of fibres 301 embedded in a matrix 302.

Figure 6 shows another layer 30d. This layer 30d is similar to the layer 30c shown in Figure 5. The difference is that the matrix 302 in layer 30d has two portions 303 which do not have any reinforcing fibres; these two portions 303 are only made of the matrix's material.

The matrix's material in the case of Figures 5 and 6 has the corresponding high friction and low adhesion properties.

A preferred embodiment of the element 1 of the invention, example II, having the flexible layers 30c or 30d of Figures 5 or 6 includes six layers, each layer having a thickness of 250 µm. Each layer is made of fibreglass (FG) fabric of 204 g/mm², embedded in a matrix made of thermoplastic polyurethane (of Epurex® 4201 AU). The fibre ratio is 73%. The resulting element allows switching between a rigid state with a Young's modulus of 2876 MPa (obtained at a negative pressure of -0.86 bar) to a flexible state with a Young's modulus of 84 MPa (measured at atmospheric pressure). The Young's modulus was obtained for a strain of 0.2%-0.4%.

Figure 7 shows still another possible embodiment of layers constituting the laminar structure of the element.

The layer 30e in Figure 7 is similar to those of Figures 5 or 6, but in this case the matrix 302 reinforced with a plurality of unidirectional non-woven fibres 301 forms a core, which is further coated by coatings 41, 42 both made of a same material having high friction and low adhesion.

The fibres in the layers of figures 5-7 are unidirectional non-woven fibres. But it is also possible that the fibres are multidirectional or fibres forming part of a woven fabric.

The fibres 301 in the embodiments shown Figures 5-7 can be any of the following: glass fibres, carbon fibres, aramid fibres or polyester fibres. The material of the matrix 302 can be a thermostable polymer, such as epoxy, polyester, polyuria, vinylester, phenolic, polyimide, polyamide resins, or a thermoplastic polymer, such as ABS, PP, PEHD, PEEK, PVC, PU, etc.

In the following examples the friction coefficient between surfaces made of a PUR-resin (Polyol) with a PUR-hardener (Isocyanate) has been determined with a sled of metal block 60x54 mm, weighing 268 g, and a pre-load of 0.2 N. The rest of the conditions used during testing of the materials are those described in the standard ASTM 1894:
* Polyurea (RAKU-TOOL® PC-3411 resin with RAKU-TOOL® PH-3911 Isocyanate from RAMPF®), reinforced with bidirectional carbon fibre fabric (200 g/m²). Its friction coefficient was measured to be 2.25.
* Polyurea (RAKU-TOOL® PC-3411 resin with RAKU-TOOL® PH-3911 Isocyanate from RAMPF®), reinforced with bidirectional glass fibre fabric (204 g/m²). Its friction coefficient was measured to be 2.20.

These two examples are used to measure the friction coefficient of the polyureas, in the layer 30c where the surfaces are made with the same material as the matrix 302 reinforced with fibres 301 (Fig. 5).

When the element 1 is used as an orthopaedic device, it is capable to adapt to the individual shape of the limb of the patient. In its flexible state the element 1 is adapted to the shape of the limb, and when vacuum is applied the element 1 locks to its rigid state to provide support and stabilization. For this purpose, it is important to have a high stiffness ratio between the flexible and rigid states in addition to each layer preferably being made of a material with a high Young's modulus. In an ideal case, the layers 30, when in the rigid state, are completely stuck to each other through the applied negative pressure, the stiffness of the element is n² times higher than in the flexible state under atmospheric condition, n being the number of layers in the element. In the real case this stiffness-increase factor of n² is approached, depending on the actual coefficient of friction that still may allow some sliding between the layers. For example, an element having a thickness of 2.4 mm made of 6 layers of 0.4 mm each has a stiffness ratio between the rigid and flexible states of 36 (=6^2). The stiffness ratio to be achieved by the element depends on the type of application. For instance, a ratio of 4 is not sufficient in the case of orthosis fitting. For fitting orthoses, an element with twelve thin layers -and thus a ratio of 144-works. But it is also possible to double the thickness of the layers, include only six layers, and the resulting element is sufficiently flexible and is able to reach a similar stiffness suitable for orthosis applications.

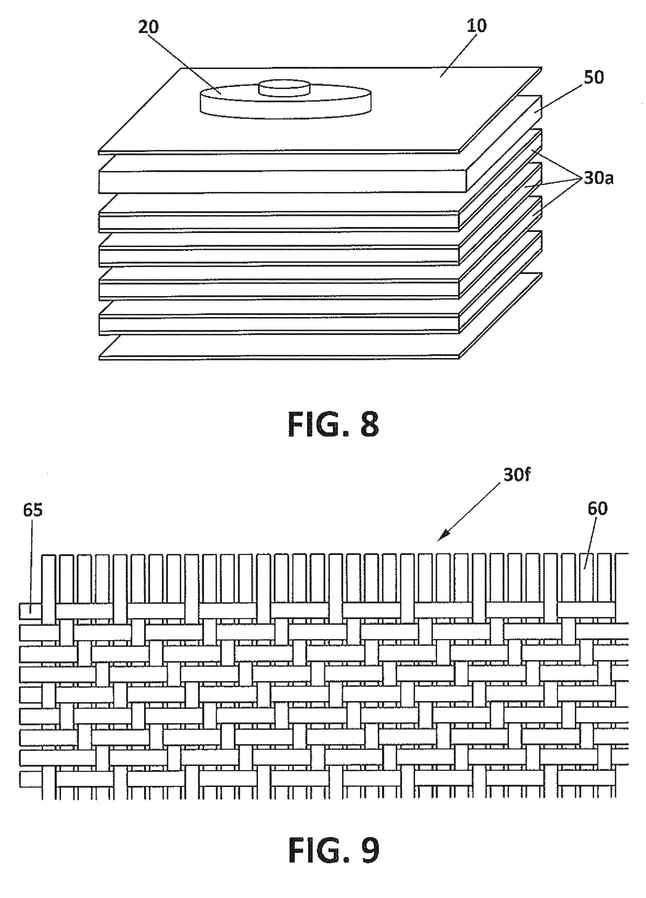

In order to apply a homogenous force during the compression of the different layers, as shown in Figure 8, the element 10 further comprises an air permeable layer 50, such as a netted structure, inserted parallel with the layers 30a inside the flexible envelope 10. The air permeable layer 50 allows the vacuum to be uniformly distributed. For example, a plastic mesh made with fibre of 100 µm diameter and open cells around 3x3mm, provides a uniform pressure distribution.

The valve 20 is inserted into the envelope 10 on the side next to the air permeable layer 50. This avoids the blocking of the airflow by a layer 30a that gets stuck to the valve orifice. Additionally the air permeable layer 50 prevents the outer layer from sticking to the envelope which could lead to loss of flexibility in the flexible state.

As indicated above, it is desirable that the layers are made of material with a high Young's modulus to make an element with a high stiffness state; but materials having a high Young's modulus usually have low extensibility. Because they are not extensible they cannot fit every 3D shape. In order to fit any 3D form or shape, especially the ones with irregular surfaces, in another possible embodiment of the invention (as shown in Figure 9), the layer 30f is provided in the form of ribbons or tapes woven to add degrees of freedom to the layer. To keep this structure organized after repeated uses and avoid overlaps and loss of the ribbons, the borders of any 2D pattern can be sewn and cut, taking care to ensure that both ends of each ribbon in the pattern have been sewn.

In order to make the tapes -the warp 60 and weft 65 yarns- that form the layer 30f, any of the flexible layers 30, 30a, 30b, 30c, 30d or 30e of the previous embodiments are cut in tapes or ribbons of the desired width which are then woven.

In the cases where the layer is a composite material made of a polymer matrix reinforced with fibres, such as the layers 30c, 30d or 30e, of Figures 5-7, it is also possible to directly make the composite of the specific width.

Making the weaving smaller, i.e. with ribbons of smaller width, allows for a better fitting. For human body fitting, 3x1 twill weaving made with a tape or ribbon having a width of 4 mm and with a 1 mm gap between the tapes/ribbons gives a good result. But any ribbon's width can be used depending on the purpose.

The tapes or ribbons can be made with a fibreglass roving of 600tex (from PPG) flatted at 4 mm width. The fibre rovings are then impregnated with thermoplastic polyurethane (such as Elastollan® 890 A10 from BASF), respecting a volume ratio matrix/fibre around 30/70. In this case, the surface roughness should be around 1.27 to achieve the right friction and tackiness properties of the first and surfaces.

It is also possible to make a smaller weaving using a fibre glass roving of 300tex (from PPG) flatted at 2.5 mm width.

A preferred embodiment of the element 1 of the invention, example III, having the flexible layers 30f shown in Figure 9 includes six layers, each layer having a total thickness of 450 µm. Each layer is made of ribbons or tapes, having a thickness of 160 µm, woven in a 3x1 twill. The ribbons are made with fibreglass (FG) rovings of 600tex are embedded in a matrix made of thermoplastic polyurethane of Epurex® 4201 AU (or fibreglass-reinforced TPU). The fibre ratio in this case is 60%. The resulting element allows switching between a rigid state with a Young's modulus of 546 MPa (obtained at a negative pressure of -0.86 bar) to a flexible state with a Young's modulus of 24 MPa (measured at atmospheric pressure). The Young's modulus was obtained for a strain of 0.2%-0.4%.

Regarding the weavings, many solutions are possible: plain, five-point harness, 16-point harness, 2x2 twill or 3x1 twill.

Twill weaving has the advantage of being more flexible and drapeable than plain weaving. Harness weaving is also a good option regarding drapeability.

In any case, independently of the weaving used (plain, twill, harness, ..) in the preferred embodiment there is a gap -of 1 mm approximately- between each ribbon in both the warp and the weft directions, in order to allow some degree of freedom between separate ribbons and thereby obtain enough drapeability to fit the human body.

The following Table 2 synthesizes the main features of the three examples I, II, III previously given for the layers inside the element, and their properties. In the three examples the layers of the element are enclosed in an airtight bag of PP/HDPE. The element further contains a nylon mesh for the repartition of the vacuum, adding a total of 0.6 mm to the thickness of the element.

**Table 2**

| Ex | Specification | Fibre ratio (%) | Static Friction Coeff. | Tack Stress (MPa) (N/mm²) | Tack Energy (J/m²) | Young's mod. - rigid (MPa) (at -0.86 bar) | Young's mod - flexible (MPa) (at atmosph. pressure) |
|---|---|---|---|---|---|---|---|
| I | 37 layers of TPU Epurex® 4201 AU | No fibers | 1.41 | -0.04 | 0.85 | 167 | 0.12 |
| II | 6 layers of FG fabric (204 g/m²) in a matrix of TPU Epurex® 4201 AU | 73% FG | 7.59 | -0.03 | 2.27 | 2876 | 84 |
| III | Ribbon woven 3x1 twill 6 layers FG U600tex, in a matrix of TPU Epurex® 4201 AU | 60% FG | 4.50 | -0.03 | 1.65 | 546 | 24 |

In this text, the term "comprises" and its derivations (such as "comprising", etc.) should not be understood in an excluding sense, that is, these terms should not be interpreted as excluding the possibility that what is described and defined may include further elements, steps, etc.

In the context of the present invention, the term "approximately" and terms of its family (such as "approximate", etc.) should be understood as indicating values very near to those which accompany the aforementioned term. That is to say, a deviation within reasonable limits from an exact value should be accepted, because a skilled person in the art will understand that such a deviation from the values indicated is inevitable due to measurement inaccuracies, etc. The same applies to the terms "about" and "around" and "substantially".

## Claims

1. Element (1) with variable stiffness controlled by negative pressure, the element comprising:
- a gas-tight envelope (10);
- a plurality of flexible layers (30, 30a, 30b, 30c, 30d, 30e) in the envelope, each layer (30, 30a, 30b, 30c, 30d, 30e) having a first surface (31, 41) and a second surface (32, 42); and,
- a valve (20) adapted to evacuate the interior of the envelope (10);
**characterised in that**:
- the first and second surfaces (31, 41, 32, 42) of two adjacent layers have a friction coefficient between each other which is higher than 0.5;
- the first and second surfaces (31, 41, 32, 42) of two adjacent layers have adhesion properties, such that a normal force per unit area of below 0.07 N/mm² is required to separate them and/or the energy per unit area required to separate them in normal direction is below 6.7 J/m².

2. Element (1) according to claim 1, wherein the layers (30, 30f) are made of a single material.

3. Element (1) according to claim 1, wherein the layers (30c, 30d, 30e, 30f) are made of a plurality of fibres (301) embedded in a matrix (302).

4. Element (1) according to claim 3, wherein the fibres (301) are unidirectional.

5. Element (1) according to claim 3, wherein the fibres (301) are bidirectional or multidirectional.

6. Element (1) according to any of claims 3-5, wherein the fibres (301) are selected from fibre glass, carbon, aramid or polyester fibres.

7. Element (1) according to any of claims 3-6, wherein the matrix (302) is made of a thermostable polymer or a thermoplastic polymer.

8. Element (1) according to any of claims 1-7, wherein each layer (30a, 30b, 30d) further comprises a core (40) coated by at least one coating (41, 42) on one side of the core (40).

9. Element (1) according to any of claims 1-7, wherein each layer (30a, 30d) further comprises a core (40) coated by respective first and second coatings (41, 42), one coating on each side of the core (40).

10. Element (1) according to any of claims 8-9, wherein the coatings (41, 42) are made of a thermoplastic polyurethane elastomer.

11. Element (1) according to any of claims 1-10, wherein the layers (30a, 30b, 30c, 30d, 30e) are made of a continuous sheet.

12. Element (1) according to any of claims 1-4 and 6-10 when dependent upon claim 4, wherein the layer (30f) is made of a woven structure of tapes (60, 65).

13. Element (1) according to any previous claim, wherein the adhesion properties of the layers are measured using an adapted probe-tack test with a pre-load equivalent to the atmospheric pressure and a waiting pre-load time of 100 s.

14. Element (1) according to claim 12, wherein the adhesion properties of the layers are measured using an adapted probe-tack test with a pre-load equivalent to the atmospheric pressure and a waiting pre-load time of 100 s, such measurements being carried out on a determined number of tapes (60, 65) placed adjacent to each other over a flat surface.

15. Element (1) according to any previous claim, wherein the adhesion properties of the layers are measured on layers as they are in use.

## Patentansprüche

1. Element (1) mit variabler Steifigkeit, die durch Unterdruck gesteuert wird, wobei das Element Folgendes aufweist:
- eine gasdichte Umhüllung (10);
- eine Vielzahl von flexiblen Schichten (30, 30a, 30b, 30c, 30d, 30e) in der Umhüllung, wobei jede Schicht (30, 30a, 30b, 30c, 30d, 30e) eine erste Fläche (31, 41) und eine zweite Fläche (32, 42) aufweist; und,
- ein Ventil (20), das geeignet ist, das Innere der Umhüllung (10) zu evakuieren;
**dadurch gekennzeichnet, dass**:
- die erste und die zweite Oberfläche (31, 41, 32, 42) von zwei benachbarten Schichten einen Reibungskoeffizienten zwischen einander aufweisen, der höher als 0,5 ist;
- die erste und die zweite Oberfläche (31, 41, 32, 42) von zwei benachbarten Schichten Hafteigenschaften aufweisen, so dass eine Normalkraft pro Flächeneinheit von unter 0,07 N/mm² erforderlich ist, um sie zu trennen und/oder die Energie pro Flächeneinheit, die benötigt wird, um sie in der normalen Richtung zu trennen, unter 6,7 J/m² ist.

2. Element (1) nach Anspruch 1, wobei die Schichten (30, 30f) aus einem einzigen Material hergestellt sind.

3. Element (1) nach Anspruch 1, wobei die Schichten (30c, 30d, 30e, 30f) aus einer Vielzahl von in einer Matrix (302) eingebetteten Fasern (301) hergestellt sind.

4. Element (1) nach Anspruch 3, wobei die Fasern (301) unidirektional sind.

5. Element (1) nach Anspruch 3, wobei die Fasern (301) bidirektional oder multidirektional sind.

6. Element (1) nach einem der Ansprüche 3 - 5, wobei die Fasern (301) aus Glasfaser, Kohlenstoff, Aramid oder Polyesterfasern ausgewählt sind.

7. Element (1) nach einem der Ansprüche 3 - 6, wobei die Matrix (302) aus einem thermostabilen Polymer oder einem thermoplastischen Polymer hergestellt ist.

8. Element (1) nach einem der Ansprüche 1- 7, wobei jede Schicht (30a, 30b, 30d) ferner einen Kern (40) aufweist, der durch wenigstens eine Beschichtung (41, 42) auf einer Seite des Kerns (40) beschichtet ist.

9. Element (1) nach einem der Ansprüche 1- 7, wobei jede Schicht (30a, 30d) ferner einen Kern (40) aufweist, der durch entsprechende erste und zweite Beschichtungen (41, 42) beschichtet ist, wobei auf jeder Seite des Kern (40) eine Beschichtung ist.

10. Element (1) nach einem der Ansprüche 8 - 9, wobei die Beschichtungen (41, 42) aus einem thermoplastischen Polyurethan-Elastomer hergestellt sind.

11. Element (1) nach einem der Ansprüche 1 - 10, wobei die Schichten (30a, 30b, 30c, 30d, 30e) aus einem kontinuierlichen Bogen hergestellt sind.

12. Element (1) nach einem der Ansprüche 1 - 4 und 6 - 10, wenn abhängig von Anspruch 4, wobei die Schicht (30f) aus einer gewebten Struktur aus Bändern (60, 65) hergestellt ist.

13. Element (1) nach einem der voranstehenden Ansprüche, wobei die Hafteigenschaften der Schichten mittels eines angepassten probe-tack-Tests bei einer Vorbelastung entsprechend dem Atmosphärendruck und einer Warte-Vorlastzeit von 100 s gemessen werden.

14. Element (1) nach Anspruch 12, wobei die Hafteigenschaften der Schichten mittels eines angepassten probe-tack-Tests bei einer Vorbelastung entsprechend dem Atmosphärendruck und einer Warte-Vorlastzeit von 100 s gemessen werden, wobei solche Messungen an einer bestimmten Anzahl von Bändern (60, 65), die über eine flache Oberfläche nebeneinander gelegt werden, durchgeführt werden.

15. Element (1) nach einem der voranstehenden Ansprüche, wobei die Hafteigenschaften der Schichten an den Schichten gemessen werden, während sie in Gebrauch sind.

## Revendications

1. Élément (1) à rigidité variable commandée par pression négative, l'élément comprenant :
- une enveloppe étanche aux gaz (10) ;
- une pluralité de couches flexibles (30, 30a, 30b, 30c, 30d, 30e) dans l'enveloppe, chaque couche (30, 30a, 30b, 30c, 30d, 30e) ayant une première surface (31, 41) et une deuxième surface (32, 42) ; et
- une valve (20) adaptée pour évacuer l'intérieur de l'enveloppe (10) ;
**caractérisé en ce que**
- la première et la deuxième surfaces (31, 41, 32, 42) de deux couches adjacentes ont un coefficient de friction entre elles qui est supérieur à 0,5 ;
- la première et la deuxième surfaces (31, 41, 32, 42) des deux couches adjacentes ont des propriétés d'adhérence telles qu'une force normale par unité de surface inférieure à 0,07 N/mm² est nécessaire pour les séparer et/ou l'énergie par unité de surface requise pour les séparer dans la direction normale est inférieure à 6,7 J/m².

2. Élément (1) selon la revendication 1, dans lequel les couches (30, 30f) sont réalisées en un matériau unique.

3. Élément (1) selon la revendication 1, dans lequel les couches (30c, 30d, 30e, 30f) sont faites d'une pluralité de fibres (301) noyées dans une matrice (302).

4. Élément (1) selon la revendication 3, dans lequel les fibres (301) sont unidirectionnelles.

5. Élément (1) selon la revendication 3, dans lequel les fibres (301) sont bidirectionnelles ou multidirectionnelles.

6. Élément (1) selon l'une quelconque des revendications 3 à 5, dans lequel les fibres (301) sont choisies parmi les fibres de verre, les fibres de carbone, les fibres d'aramide ou les fibres de polyester.

7. Élément (1) selon l'une quelconque des revendications 3 à 6, dans lequel la matrice (302) est faite d'un polymère thermostable ou d'un polymère thermoplastique.

8. Élément (1) selon l'une quelconque des revendications 1 à 7, dans lequel chaque couche (30a, 30b, 30d) comprend en outre une âme (40) recouverte d'au moins un revêtement (41, 42) sur un côté de l'âme (40).

9. Élément (1) selon l'une quelconque des revendications 1 à 7, dans lequel chaque couche (30a, 30d) comprend en outre une âme (40) recouverte d'un premier et d'un deuxième revêtements (41, 42) respectivement, un revêtement de chaque côté de l'âme (40).

10. Élément (1) selon l'une quelconque des revendications 8 à 9, dans lequel les revêtements (41, 42) sont faits d'un élastomère de polyuréthane thermoplastique.

11. Élément (1) selon l'une quelconque des revendications 1 à 10, dans lequel les couches (30a, 30b, 30c, 30d, 30e) sont constituées d'une feuille continue.

12. Élément (1) selon l'une quelconque des revendications 1 à 4 et 6 à 10 lorsqu'elles dépendent de la revendication 4, dans lequel la couche (30f) est constituée d'une structure tissée à partir de bandes (60, 65).

13. Élément (1) selon l'une quelconque des revendications précédentes, dans lequel les propriétés d'adhérence des couches sont mesurées en utilisant un test de pégosité (probe track) adapté avec une précontrainte équivalente à la pression atmosphérique et une durée d'attente de précontrainte de 100 s.

14. Élément (1) selon la revendication 12, dans lequel les propriétés d'adhérence des couches sont mesurées en utilisant un test de pégosité (probe track) adapté avec une précontrainte équivalente à la pression atmosphérique et une durée d'attente de précontrainte de 100 s, de telles mesures étant réalisées sur un nombre déterminé de bandes (60, 65) placées les unes à côté des autres sur une surface plane.

15. Élément (1) selon l'une quelconque des revendications précédentes, dans lequel les propriétés d'adhérence des couches sont mesurées sur des couches telles qu'elles sont utilisées.
